# EUROPEAN PATENT APPLICATION

(11) **EP 2 236 095 A2**
(43) Date of publication of application: **06.10.2010**
(21) Application number: 10006454.2
(22) Date of filing: 16.03.2007
(51) Int. Cl.: A61B 17/06

(54) **Steel suture package**

(30) Priority: 04.04.2006 US 397170
(62) Divisional of application: 07005466.3
(71) Applicant: Tyco Healthcare Group LP, North Haven, CT 06473 (US)
(72) Inventor: Malinowski, Stan, Guilford, CT 06437 (US); Engebretsen, Grace E., Chicago, IL 60611 (US)
(74) Representative: Soames, Candida Jane

(57) **Abstract**

A suture package comprising: an outer envelope; a container including: a hollow tube, at least one removable end cap, the container being configured and dimensioned to house a plurality of surgical steel sutures therein; and an inner retainer having a plurality of support elements adapted to releasably retain the container therein.

## Description

### BACKGROUND

### Technical Field

The present disclosure relates generally to packaging for storing and dispensing surgical devices, and more particularly, to a package for storing and dispensing steel sutures whereby the steel sutures are retained within a container.

### Background of Related Art

A number of packages and packaging methods have been proposed over the years for packaging and delivering sutures to a surgeon in a sterile condition. However, packaging of steel surgical sutures has posed particular problems because of their size, stiffness and manner of their packaging. Stainless steel sutures require that they be secured properly to prevent displacement within the packaging, bending or kinks within the steel filament, as well as being simply and quickly removable from the packaging for use by the surgeon.

Monofilament stainless steel sutures have extremely high tensile strength relative to the diameter of the material. When implanted into tissue, stainless steel sutures will not split spontaneously and are capable of being twisted to very high tightness approaching the maximum tensile strength of the material. Stainless steel sutures have been packaged in long cardboard envelopes contained within a sterile sealable breather pouch. A continuing need exists for improved suture packaging and methods for containing steel sutures.

Therefore, it is desirable to package stainless steel sutures in a manner where they are sufficiently separated for ready access to the user when they are needed and to prevent kinks from forming in the filament. Moreover, the suture packages must be capable of receiving and holding sutures of various sizes while generally not affecting the quality, shape or strength of the suture in any way.

### SUMMARY

Accordingly, a suture package in accordance with the present disclosure includes an outer envelope and an inner retainer with four C-shaped slits equally spaced apart to releasably retain a hollow tube container therein, the container configured and dimensioned to retain steel sutures therein.

In another embodiment, the method of assembling the suture package for storing and dispensing steel sutures therein in accordance with the present disclosure includes providing a hollow tube container with one end cap, placing steel sutures within the container and capping the open end to enclose the sutures therein, providing an inner retainer with four C-shaped slits equally spaced apart whereas the container is placed through the slits in a manner for releasably securing the container within the inner retainer and providing a sterilized outer envelope to enclose the inner retainer within.

### BRIEF DESCRIPTION OF THE DRAWNGS

Although specific embodiments of the present disclosure will now be described with reference to the drawings, it should be understood that such embodiments are by way of example only and merely illustrative of but a small number of the many possible specific embodiments which can represent applications of the principles of the present disclosure. Various changes and modifications obvious to one skilled in the art to which the present disclosure pertains are deemed to be within the spirit, scope and contemplation of the present disclosure as further defined in the appended claims.
FIG. 1 is a top view of a stainless steel suture package;
FIG. 2 is an exploded perspective view of the elements which make up the stainless suture package of FIG.1; and
FIG. 3 is an exploded perspective view of a container adapted to retain steel sutures with a pair of end caps.

### DETAILED DESCRIPTION

In general, the present disclosure provides a suture package and packaging method which ensures rapid access to sutures, economically and efficiently contained within a hollow tube container to ensure containment, easier handling and storing of the steel sutures with reduced risk of compromising the sterility of the steel sutures beyond its sterilized field. Each suture is readily disposed in an advantageous, readily accessible position in the package, to be grasped with minimum possibility of pulling unwanted sutures from the package, or the need to fumble through a plurality of steel sutures to ensure that a single suture is obtained. Moreover, the manner in which the steel sutures are supplied in accordance with the present disclosure ensures that the sutures provided are in sterile condition, and without kinks or other deformities which might adversely affect their strength.

Once the sutures are assembled in the package of the present invention, the sterile package is overwrapped with a sterile barrier such as a Tyvek® commercially made by DuPont (pouch, which is impervious to germs, and which will have sufficient strength to be handled and stored without loss of sterility of the interior pack, and one which can be easily opened during transfer to the sterile field of an operation.

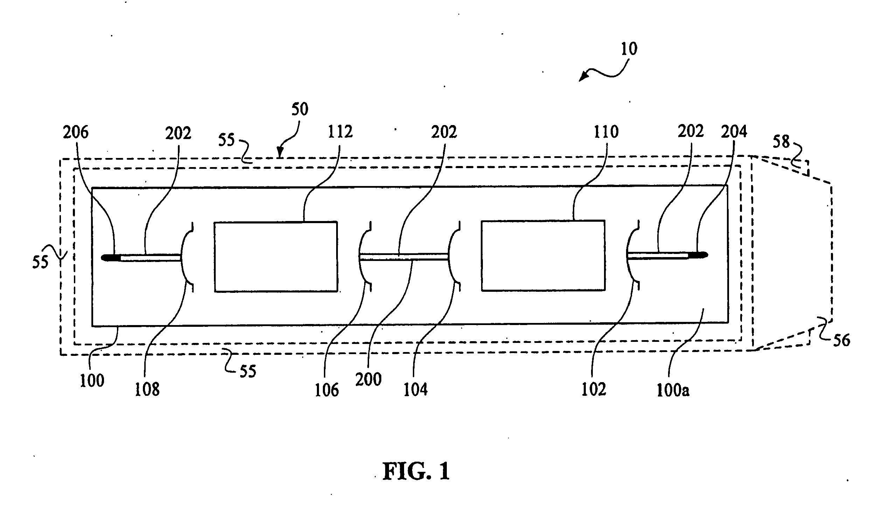

Referring now to FIG. 1, one illustrative embodiment of a suture package constructed in accordance with the present disclosure is shown as steel suture package 10 which includes an outer envelope 50, an inner retainer100, and a container 200.

The outer envelope 50 includes a pair of sheets 52 and 54 which form a sterility barrier. Sheet 52 may be formed of a suitable sterile barrier packaging material such as the commercially manufactured Tyvek®. Sheet 54 may be formed of a transparent or translucent material such as for example a clear polyethylene sheet. The two sheets are adhered together on three sides by a peripheral line of peelable adhesive 55. To make the fourth side of the seal, the adhesive 55 spans the package 10 in a manner that creates two flaps 56, 58 by which the two sheets of the outer envelope 50 can be readily pulled apart.

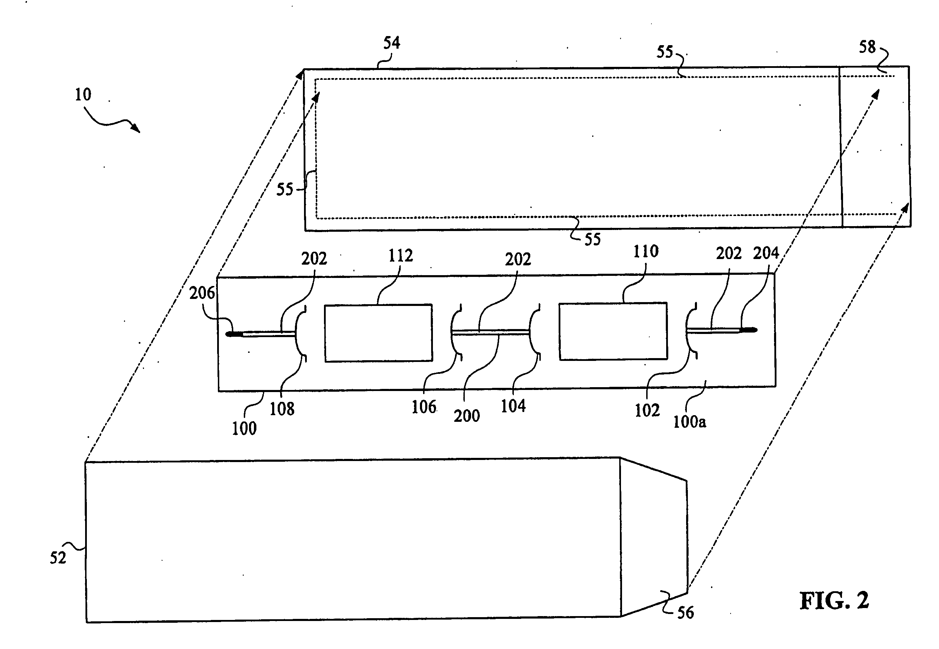

Referring to FIGS. 1 and 2, an inner retainer 100 is provided and includes a front side 100a, a rear side (not shown), four generally C-shaped slits 102, 104, 106, 108 for releasably securing container 200 to retainer 100, and two labels 110, 112. Retainer 100 may be formed of a suitable pliable material such as paper stock. The four slits 102, 104, 106, 108 are equally spaced along inner retainer 100. Two labels 110, 112 are located on the front side 100a of inner retainer 100 one between the first and second slits 102, 104 and one between the third and fourth slits 106, 108, respectively.

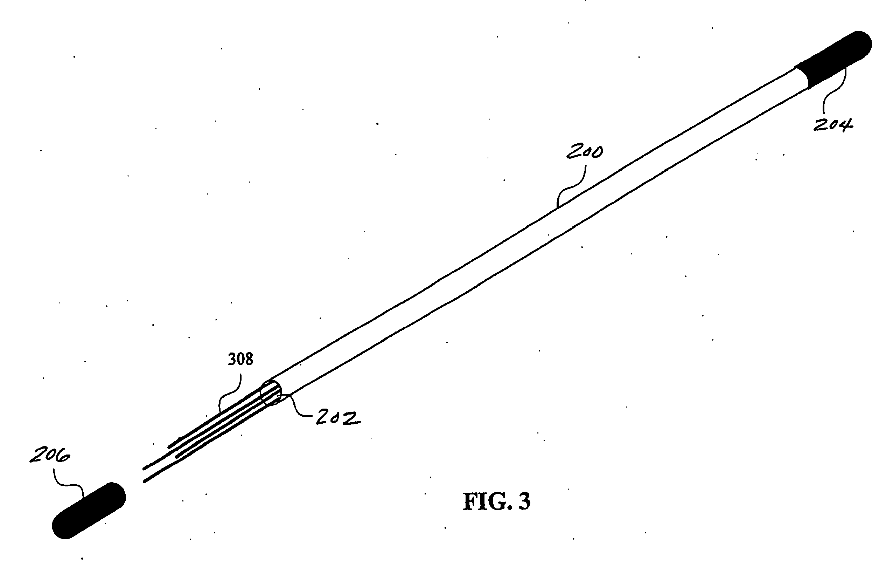

Referring to FIG. 3, in order to enclose a plurality of steel sutures 308, a container 200 is provided and includes a hollow tube 202 and a pair of end-caps 204, 206. End-caps 204, 206 are custom fit to be positioned over opposite ends of hollow tube 202 to enclose steel sutures 308 within container 200. Container 200 may be formed of any suitable material such as plastic or metal. Hollow tube 202 is configured and dimensioned so that it can hold a plurality of steel sutures 308 therein. End caps 204, 206 can be composed of a traditional rubber or thermoplastic material known to be used in sealing sterile vials, intravenous bags, catheters, drug ampules or blood bags. Alternatively, end caps 204, 206 may be composed of hydrophobic, hydrophilic or a combination of hydrophobic and hydrophilic materials. Suitable end caps can be made of any size, shape or dimension to fit a hollow tube's size, shape or dimension.

To assemble the package, one end-cap 204 is placed on one end of hollow tube 202. Steel sutures 308 are then placed into the other, open end of hollow tube 202. Once sutures 308 are all within hollow tube 202, the second end-cap 206 is placed on the open end of hollow tube 202 to enclose sutures 308 within storage container 200. Once end caps 204, 206 are placed onto container 200 retaining the sutures 308, the steel sutures 308 are sterilized using methods generally known by those skilled in the art. For example, some sterilization techniques include gamma irradiation, electron beam (E beam), treatment with ethylene oxide, or other chemical or physical treatments providing sterilization.

Container 200 is releasably secured within inner retainer 100 by the four slits 102, 104, 106, 108. Container 200 enters first slit 102 from the front side 100a of inner retainer100 and passes underneath label 110 before entering the second slit 104 from the rear side of inner retainer100. From there, container 200 proceeds along the front side 100a of inner retainer 100 before entering the third slit 106 and passing underneath label 112 before entering the fourth slit 108 from the rear side of inner retainer 100 and ending on the top side 100a of inner retainer 100.

The inner retainer 100, with container 200 releasably secured therein, is sealed between the two sheets of the outer envelope 50. The outer envelope 50 can be any conventional envelope for medical devices manufactured from any suitable material known to those skilled in the art. In one embodiment, outer envelope 50 is formed by heat sealing two panels of aluminum foil coated on the interior surfaces thereof with a heat sealable polymeric composition. The envelope is bonded around the periphery of the inner sealable pouch as illustrated in FIG. 1. Other means for sealing the outer envelope may be employed as are well known to those skilled in the art.

In another embodiment, outer envelope 50 may be formed from a hydrophobic material. The term "hydrophobic", as described herein, refers to materials that are not normally water soluble and absorb relatively low amounts of water, i.e., less than about 10% by weight. Some examples of these materials include, but are not limited to, polymers, copolymers, homopolymers, and block copolymers formed from monomers such as ε-caprolactone, glycolide, 1-lactide, d,1-lactide, d-lactide, meso-lactide, trimethylene carbonate, 4,4-dimethyl-1,3-dioxan-2-one, p-dioxanone, dioxepanone, δ-valerolactone, β-butyrolactone, ε-decalactone, 2,5-diketomorpholine, pivalolactone, α,α-diethylpropiolactone, 6,8-dioxabicyclooctan-7-one, ethylene carbonate, ethylene oxalate, 3-methyl-1,4-dioxane-2,5-dione, 3,3-dimethyl-1,4-dioxane-2,5-dione, and other subtituted glycolides, and substituted lactides. Some additionally useful hydrophobic materials include polyolefins (i.e. Tyvek.®) and polysiloxanes.

In another embodiment, outer envelope 50 may be formed from a hydrophilic material. The term "hydrophilic", as described herein, refers to materials that are normally water soluble and absorb relatively high amounts of water. Some examples of these materials include, but are not limited to, polyalkylene glycols, such as polyethylene glycol, polyacrylates such as polymers of methacrylates and 2-hydroxyethyl methylacrylate, aminoalkyl acrylates, such as N,N-dimethylacrylamide, polyvinylalcohols, polyvinylpyrrolidones, polyoxyethylenes, polyacrylamides, poly(2-hydroxy-ethylmethacrylate), polymethacrylamide, dextran, alginic acid, sodium alginate, polysaccharides, gelatine and copolymers of two or more of the monomers from which the above polymers are derived and polyoxyethylene/polyoxypropylene block copolymers.

In still another embodiment, outer envelope 50 can be made of a combination of hydrophobic and hydrophilic materials. An example of an outer envelope with a combination of hydrophobic and hydrophilic materials can include a polyolefin sheet (i.e. Tyvek.®) and a polyethylene sheet adhered together with a release agent.

When the suture is to be used, it is easily removed from the package by pulling off an end cap and grasping one end of the suture and pulling the suture upwardly. The removal of the suture is greatly facilitated by the hollow tube opening. For example, the surgeon or surgical nurse can invert the tube slightly to displace a surgical suture from the hollow tube and pull the suture out of the package without a mass of sutures falling out into the non-sterile field. Thus, the possibility of tangling the sutures or pulling more than one suture out of the package is avoided. The process of getting the suture to the surgeon, with the suture ready for application to the patient without having to re-sterilize or having to obtain a new suture due to a deformity in the suture, is vastly simplified and corrected, compared to prior approaches.

Those skilled in the art will readily appreciate a variety of embodiments once acquainted with the present disclosure, all of which are intended to be included within the scope of the following claims.

## Claims

1. A suture package comprising:
an outer envelope;
a container including:
a hollow tube,
at least one removable end cap,
the container being configured and dimensioned to house a plurality of surgical steel sutures therein; and
an inner retainer having a plurality of support elements adapted to releasably retain the container therein.

2. The suture package of claim 1, wherein the outer envelope includes a first sheet and a second sheet having top and bottom transverse edges and longitudinal side edges adhered together and at least one peelable flap on a top edge of the seal for permitting access to the container.

3. The suture package of claim 2, wherein the first sheet is a fibrous layer.

4. The suture package of claim 2 or 3, wherein the second sheet is a plastic layer.

5. The suture package of claim 4, as dependent on claim 3, wherein the at least one peelable flap includes a release agent to facilitate separation of said plastic layer from said fibrous layer to open said package.

6. The suture package of claim 5, wherein the release agent comprises an adhesive.

7. The suture package of any one of the preceding claims, wherein the support members of the inner retainer includes a front side and a rear side, said front side comprising four C-shaped slits equally spaced along said inner retainer for securing the container.

8. The suture package of any one of the preceding claims, wherein the inner retainer includes two labels on the front side of the inner retainer between first and second slits and between third and fourth slits.

9. A method for packaging surgical steel sutures comprising the steps of:
providing an outer envelope;
providing a container including:
a hollow tube,
at least one removable end cap to house a plurality of surgical steel sutures, and
providing an inner retainer having a plurality of support elements adapted to releasably retain the container therein;
placing a first end cap on a distal opening end of the hollow tube container;
placing steel sutures into a proximal opening of the hollow tube container;
placing a second end cap on the proximal opening to enclose the steel sutures within the container;
securing the container to the inner retainer; and
enclosing the inner retainer within the outer envelope.

10. The method as in claim 9, further comprising the step of sterilizing the steel suture package while the end caps are in place on the open ends of the container.

11. The method as in claim 9 or 10, wherein the step of securing the container to the inner retainer further comprises placing the container within four equally spaced apart C-shaped slits.

12. The method as in claim 9, 10 or 11, wherein the step of enclosing the inner retainer within the outer envelope comprises a first fibrous sheet and a second plastic sheet of the outer envelope sealed along four edges by a release agent.

13. The method as in claim 12, wherein the release agent is an adhesive.

14. The method as in clam 12 or 13, wherein the fibrous sheet comprises a polyolefin and the plastic sheet comprises a polyethylene.

15. The method as in any one of claims 9 to 14, wherein the step of securing the container within the inner retainer further comprises:
passing the container through the first slit form the front side of the inner retainer;
passing the container underneath the first label and entering the second slit from the rear side of the inner retainer;
passing the container along the front side of the inner retainer and entering the third slit passing underneath the second label and entering the fourth slit from the rear side on the inner retainer and ending on the front side of the inner retainer.

16. A steel suture kit comprising:
an outer envelope:
a container disposed within the outer envelope, the container including:
a hollow tube, and
at least one removable end cap,
a plurality of surgical steel sutures disposed within the hollow tube; and
an inner retainer having a plurality of support elements adapted to releasably retain the container therein.
